# EUROPEAN PATENT APPLICATION

(11) **EP 4 459 192 A1**
(43) Date of publication of application: **06.11.2024**
(21) Application number: 22913854.0
(22) Date of filing: 15.11.2022
(51) Int. Cl.: F24C 15/20, A61L 9/015, A61L 9/20

(54) **PURIFICATION DEVICE AND RANGE HOOD**

(30) Priority: 31.12.2021 CN 202111672926; 31.12.2021 CN 202123455201 U
(71) Applicant: Guangdong Arcair Appliance Co., Ltd., Guangdong 528318 (CN)
(72) Inventor: KANG, Zuotian, Foshan, Guangdong 528318 (CN); TANG, Haijiang, Foshan, Guangdong 528318 (CN); LIN, Zhuojun, Foshan, Guangdong 528318 (CN)
(74) Representative: Ipey
(86) International application number: PCT/CN2022/131976
(87) International publication number: WO 2023/124595

(57) **Abstract**

A purification device (100) and a range hood are provided. The purification device (100) comprises a housing (10), a purification assembly (20) and an electrical assembly (40). The housing (10) is provided with an airflow channel (11), air inlet holes (12) and air outlet holes (13). The purification assembly (20) comprises an ozone generation assembly (22) and ultraviolet lamps (201). The ozone generation assembly (22) and the ultraviolet lamps (201) are sequentially arranged in the airflow channel (11) along an airflow direction of the airflow channel (11). The ozone generation assembly (22) is used for generating ozone. The electrical assembly (40) is mounted on the upper part of the housing (10). The electrical assembly (40) comprises lamp holders (41), and the ultraviolet lamps (201) are mounted on the lamp holders (41). The electrical assembly (40) is electrically connected to the ozone generation assembly (22), and the electrical assembly (40) is arranged on the upper part of the housing (10) so that the electrical assembly (40) can be conveniently connected to the mains supply and will not interfere with the structure around the purification device (100), thereby facilitating mounting of the purification device (100). Air purification is carried out by combining ozone and ultraviolet light and thus the purification effect is better.

## Description

### TECHNICAL FIELD

The present disclosure relates to the technical field of range hoods and in particular to a purification device and a range hood.

### BACKGROUND

The fume purifiers can perform purification on hazardous substances in the fumes to achieve sterilization and disinfection effect as well as good deodorization effect, so as to make gases discharged from the fume purifiers cleaner and safer. The working principle of the purifiers is that sterilization and deodorization are performed on fumes by using generated ozone. However, the existing purifiers do not have good purification effect and the electrical structures and other structural layouts of the purifiers are to be improved.

### SUMMARY

The present disclosure provides a purification device and range hood.

There is provided a purification device, which includes:
a housing, wherein the housing is provided with an airflow channel, air inlet holes and air outlet holes, the air inlet holes are disposed at a lower part of the housing, the air outlet holes are disposed at an upper part of the housing, and the airflow channel communicates the air inlet holes with the air outlet holes;
a purification assembly, wherein the purification assembly comprises ozone generation assemblies and ultraviolet lamps, the ozone generation assemblies and the ultraviolet lamps are sequentially arranged in the airflow channel along an airflow direction of the airflow channel, and the ozone generation assemblies are used to generate ozone; and
an electrical assembly, wherein the electrical assembly is mounted at the upper part of the housing, the electrical assembly comprises lamp holders, the ultraviolet lamps are mounted on the lamp holders, and the electrical assembly is electrically connected with the ozone generation assemblies.

In the purification device of the embodiments of the present disclosure, the ozone generation assemblies and the ultraviolet lamps cooperate to perform air purification by ozone and ultraviolet light so as to achieve better purification effect. Furthermore, the disposal of the electrical assembly on the upper part of the housing facilitates connection of the electrical assembly with the mains supply and avoids interfering with structures around the purification device to facilitate the mounting of the purification device.

In some embodiments, an opening is disposed on the top of the housing, and the electrical assembly comprises a mounting casing which partially protrudes into the airflow channel and closes the opening; the lamp holders are mounted on the mounting casing.

In some embodiments, a mounting space isolated from the airflow channel is formed in the mounting casing, the lamp holders are mounted in the mounting space, and the ultraviolet lamps protrude into the mounting space from the airflow channel and are connected with the lamp holders.

In some embodiments, the mounting casing comprises a first casing and a second casing, the first casing and the second casing are detachably connected and enclosed to form the mounting space, and the first casing is located above the second casing.

In some embodiments, the electrical assembly comprises a socket on the first casing and the socket is exposed on the top of the purification device.

In some embodiments, the housing comprises a housing body and an air inlet casing, the air inlet casing is mounted at a lower part of the housing body, the air outlet holes and a part of the airflow channel are disposed in the housing body, the air inlet holes and a part of the airflow channel are disposed in the air inlet casing, and the ozone generation assemblies are mounted inside the air inlet casing.

In some embodiments, the purification assembly comprises a cover plate, an accommodation space is formed between the cover plate and the air inlet casing, the ozone generation assemblies are located in the accommodation space, and the accommodation space is in communication with the airflow channel.

In some embodiments, plural partition strips are disposed on the cover plate, mounting holes are formed on the partition strips, each of the ozone generation assemblies comprises a mounting column and a coil surrounding the mounting column, and the mounting column is mounted on the mounting holes.

In some embodiments, the plural partition strips and the cover plate form a mounting groove, the purification assembly comprises a transformer electrically connected with the ozone generation assemblies, and the transformer is disposed inside the mounting groove.

The range hood provided by the embodiments of the present disclosure includes a host machine, the purification device of any one of the embodiments and a fan. The purification device is mounted on the host machine. The fan is connected to the purification device to suck gases and discharge the gases into the purification device.

Additional aspects and advantages of the present disclosure will be partially given in the following descriptions and partially become obvious from the following descriptions or understood by practice of the present disclosure.

### BRIEF DESCRIPTION OF DRAWINGS

The above and/or additional aspects and advantages of the present disclosure will become obvious and intelligible from the descriptions made to the embodiments by referring to the drawings.
FIG. 1 is a stereoscopic diagram illustrating a purification device according to an embodiment of the present disclosure.
FIG. 2 is a stereoscopic diagram illustrating a purification device according to an embodiment of the present disclosure.
FIG. 3 is an exploded view illustrating a purification device according to an embodiment of the present disclosure.
FIG. 4 is a stereoscopic sectional view illustrating a purification device according to an embodiment of the present disclosure.
FIG. 5 is a stereoscopic diagram illustrating a purification assembly according to an embodiment of the present disclosure.
FIG. 6 is an exploded view illustrating a purification assembly according to an embodiment of the present disclosure.
FIG. 7 is an exploded view illustrating a range hood according to an embodiment of the present disclosure.

Numerals of the drawings are described below:
100. purification device, 10. housing, 11. airflow channel, 12. air inlet hole, 13. air outlet hole, 14. housing body, 15. air inlet casing, 20. purification assembly, 201. ultraviolet lamp, 22. ozone generation assembly, 21. cover plate, 211. inner wall, 212. partition strip, 21. mounting groove, 214. through hole, 221. mounting column, 222. coil, 23. transformer, 30. accommodation space, 40. electrical assembly, 41. lamp holder, 42. mounting casing, 421. mounting space, 422. first casing, 423. second casing, 43. socket, 431. first plug fixing socket, 432. second plug fixing socket, 44. circuit board, 45. air velocity sensor, 1000. range hood, 200. host machine, and 300. fan.

### DETAILED DESCRIPTION

The embodiments of the present disclosure will be detailed below with examples illustrated in the accompanying drawings. Same or similar numerals represent same or similar elements or elements having same or similar functions throughout the specification. The embodiments described below by referring to the drawings are only illustrative, and used to explain the present disclosure and shall not be understood as limiting of the present disclosure.

In the descriptions of the present disclosure, it is understood that the orientation or positional relationship indicated by the terms such as "central", "longitudinal", "transverse", "length", "width", "thickness", "upper", "lower", "front", "rear", "left", "right", "vertical", "horizontal", "top", "bottom", "inside", "outside", "clockwise", and "counterclockwise" and the like is based on the orientation or positional relationship shown in the drawings and used only for ease of descriptions and simplification of descriptions and does not indicate or imply that the indicated devices or elements must have a particular orientation, or be constructed or operated in a particular orientation. Therefore, such terms shall not be understood as limiting of the present disclosure. Furthermore, the terms such as "first" and "second" are used only for the purpose of descriptions and shall not be understood as indicating or implying any relative importance or implicitly indicating the number of the indicated technical features. Therefore, the features defined by the "first" and "second" may explicitly or implicitly include one or more such features. In the descriptions of the present disclosure, "plural" means two or more, unless otherwise clearly stated.

In the descriptions of the present disclosure, it is noted that, unless otherwise clearly stated or defined, the terms "mount", "connect", "couple" and the like shall be understood in broad sense, for example, may be fixed connection, or detachable connection, or integral connection; or mechanical connection, or electrical connection or mutual communication; or direct connection or indirect connection through medium or internal communication of two elements or mutual interactions of two elements. Those skilled in the arts may understand the specific meanings of the above terms in the present disclosure based on specific situations.

In the present disclosure, unless otherwise clearly stated or defined, the first feature being "on" or "below" the second feature refers to that the first feature and the second feature are in direct contact, or the first feature and the second feature are not in direct contact but in indirect contact through additional features between them. Furthermore, the first feature being "above" or "on" the second feature refers to that the first feature is exactly above or obliquely above the second feature, or only refers to that the first feature has a higher horizontal height than the second feature. The first feature being "under" or "below" the second feature refers to that the first feature is exactly under or obliquely below the second feature, or only refers to that the first feature has a smaller horizontal height than the second feature.

Many different embodiments or examples are provided below to achieve different structures of the present disclosure. In order to simplify the present disclosure, the components and disposals of the specific examples below are described. Of course, they are only illustrative and not intended to limit the present disclosure. In addition, reference numerals and/or reference letters may be repeated in different examples of the present disclosure for the purpose of simplification and clarity rather than indicating the relationship of the discussed embodiments and/or disposals. Furthermore, the present disclosure provides examples of various specific processes and materials but those skilled in the arts may be aware of application or other processes and/or use of other materials.

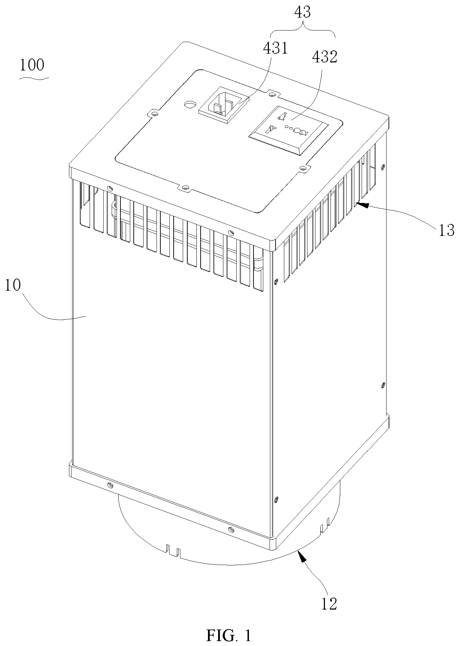

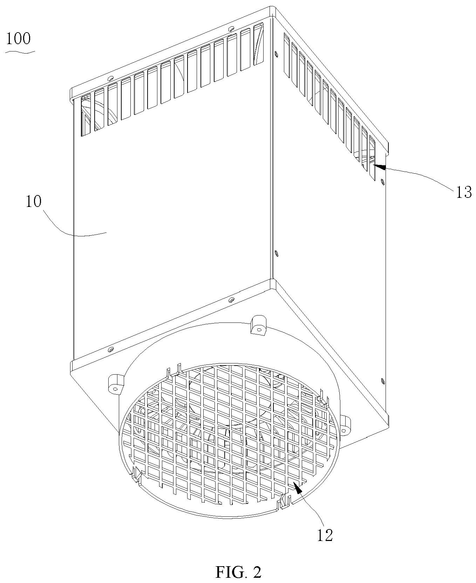

As shown in FIGS. 1 to 4, a purification device 100 in one or more embodiments of the present disclosure includes a housing 10, a purification assembly 20 and an electrical assembly 40. An airflow channel 11, air inlet holes 12 and air outlet holes 13 are disposed in the housing 10. The air inlet holes 12 are disposed on a lower part of the housing 10 and the air outlet holes 13 are disposed on an upper part of the housing 10. The airflow channel 11 communicates the air inlet holes 12 with the air outlet holes 13. The purification assembly 20 includes ultraviolet lamps 201 and ozone generation assemblies 22. The ozone generation assemblies 22 and the ultraviolet lamps 201 are sequentially arranged in the airflow channel 11 along an airflow direction of the airflow channel 11. The ozone generation assemblies 22 are used to generate ozone. The electrical assembly 40 is mounted on the upper part of the housing 10. The electrical assembly 40 includes lamp holders 41, and the ultraviolet lamps 201 are mounted on the lamp holders 41. The electrical assembly 40 is in electrical connection with the ozone generation assemblies 22.

In the purification device 100 of the embodiments of the present disclosure, the ozone generation assemblies 22 and the ultraviolet lamps 201 cooperate to perform air purification by ozone and ultraviolet light so as to achieve better purification effect. Furthermore, the disposal of the electrical assembly 40 on the upper part of the housing 10 facilitates connection of the electrical assembly 40 with the mains supply and avoids interfering with structures around the purification device 100 to facilitate the mounting of the purification device 100.

Specifically, the housing 10 may be made of plastic because the plastic is easily available and has high plasticity and thus helps mass production of the purification devices 100. In one example, the housing 10 may be made of stainless steel to facilitate cleaning for the housing 10. Therefore, no limitation is made herein for the specific material of the housing 10. Specifically, the housing 10 may be cylindrical or cuboid, which is not limited herein.

In the embodiments of the present disclosure, the air inlet holes 12 are located at a bottom end of the housing 10, and the air outlet holes 13 are located on a side surface of the housing 10. Plural air inlet holes 12 and plural air outlet holes 13 are disposed. The plural air inlet holes 12 are disposed in a spacing and the plural air outlet holes 13 are disposed in a spacing along a circumferential direction of the housing 10.

The ozone generation assemblies 22 can ionize air to form ozone after being applied with a working voltage. Ozone is a strong oxidant which can destroy and decomposing cell walls of bacteria to diffuse into cells and oxidize and decompose glucose oxidase and the like necessary for the bacteria to oxidize glucose and can also react directly with the bacteria and viruses so as to interrupt the metabolism and reproduction process of the bacteria. In addition, Ozone can oxidize various odorous inorganic or organic substances, for example, ozone can decompose odorous gases such as ammonia gas, benzene and hydrogen sulfide, so as to achieve deodorization effect. The ozone has the advantages of quick sterilization, disinfection and deodorization and strong effect.

The ultraviolet lamps 201 can generate ultraviolet light to kill the bacteria and viruses in the airflow channel 11 as well as decomposing ozone, and thus the air can be discharged indoors or outdoors after purification. The ultraviolet lamps can generate 254nm ultraviolet light.

Similar to the light emission principle of the daylight lamps and energy-saving lamps, the mercury atoms in the lamp tubes of the ultraviolet (UV) lamps are excited to generate characteristics spectral lines of the mercury and the low-pressure mercury vapor mainly generates 254nm ultraviolet light. The lamp tubes of the ultraviolet lamps 201 are made of ultraviolet-transmission glass or quartz glass and the ultraviolet light can be emitted through glass walls. The ultraviolet light with the wavelength of 254nm can be easily absorbed into organisms and act on a genetic material DNA of the organisms and then destroy the DNA, so as to bring death to the bacteria, effectively killing the bacteria. The ultraviolet light can kill the bacteria and viruses with high intensity in short time, for example, bacillus coli and mycobacterium tuberculosis can be killed in only 0.4 seconds. The ultraviolet light sterilization is a pure physical disinfection method without bringing secondary pollution.

In addition to killing the bacteria and viruses, the UV lamps can accelerate ozone decomposition with the 254nm ultraviolet light and increase the oxidation capability. After gases enter, for deodorization, a chamber of the ozone generation assemblies 22 at the lower part of the purifier housing 10 from a range hood, the gases carrying ozone can then enter the ultraviolet lamps 201, and under the synergic effect of the ultraviolet light, due to formation of hydroxyl radicals, the gases carrying ozone effectively destroy the molecular structures of the organic matters and finally decompose it. The gases with ozone decomposed can be discharged indoors or outdoors through the air outlet holes 13 on the upper part of the purification device 100.

After the above gases go through the steps of ozone deodorization, killing bacteria and viruses with ultraviolet light and decomposing ozone with ultraviolet light, the clean gases are discharged indoors or outdoors. Thus, the gases are cleaner and have no harm to human body after ozone decomposition.

The electrical assembly 40 is located on the upper part of the housing 10 such that external cables can supply power to the purification device 100 from the top of the purification device 100. In this case, disorderly external cables can be avoided and further, the external cables can be hidden when the purification device 100 is mounted in suspended way.

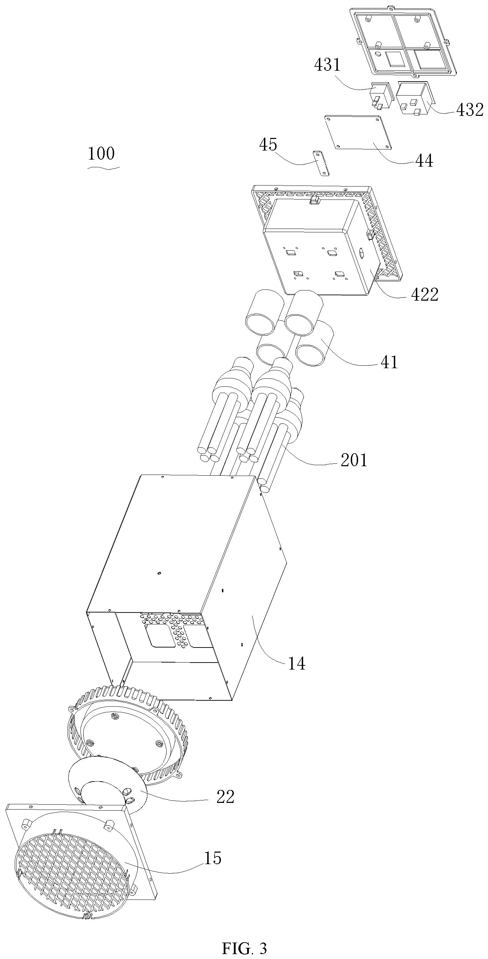

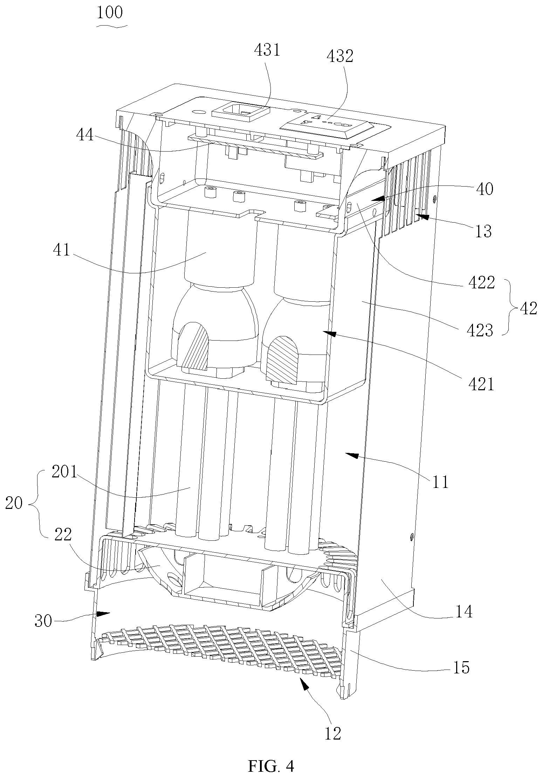

With reference to FIGS. 3 and 4, in some embodiments, the top of the housing 10 has an opening and the electrical assembly 40 includes a mounting casing 42 which partially protrudes into the airflow channel 11 and closes the opening; the lamp holders 41 are mounted on the mounting casing 42.

When the lamp holders 41 are mounted on the mounting casing 42, the lamp holders 41 can be fixed, which helps the mounting of the ultraviolet lamps 201. Specifically, the mounting casing 42 can be mounted to the housing 10 by screws to facilitate mounting and dismounting of the mounting casing 42 to and from the housing 10 and help the maintenance of the purification assembly 20. Furthermore, the lamp holders 41 can be mounted to the mounting casing 42 by screws.

With reference to FIGS. 3 and 4, in some embodiments, a mounting space 421 isolated from the airflow channel 11 is formed in the mounting casing 42, and the lamp holders 41 are located inside the mounting space 421. The ultraviolet lamps 201 protrude from the airflow channel 11 into the mounting space 421 and are connected with the lamp holders 41.

The disposal of the lamp holders 41 in the mounting space 421 prevents the gases in the airflow channel 11 from entering the mounting space 421, which ensures the safety of the electrical connection of the ultraviolet lamps 201. It should be noted that the lamp holders 41 are electrically connected to the ultraviolet lamps 201 and the lamp holders 41 can supply power from the mains supply to the ultraviolet lamps 201.

With reference to FIGS. 3 and 4, in some embodiments, the mounting casing 42 includes a first casing 422 and a second casing 423. The first casing 422 and the second casing 423 are detachably connected and enclosed to form the mounting space 421. The first casing 422 is located above the second casing 423.

The first casing 422 and the second casing 423 are detachably connected and enclosed to form the mounting space 421 such that the mounting space 421 can be easily formed and the lamp holders 41 can easily be mounted into the mounting space 421. The first casing 422 and the second casing 423 may be mounted by screws or connected another way. The lamp holders 41 may be mounted to the first casing 422 by screws.

With reference to FIGS. 3 and 4, in some embodiments, the electrical assembly 40 includes a socket 43 on the first casing 422 and the socket 43 is exposed on the top of the purification device 100.

Thus, the socket 43 can be connected with the mains supply to supply power to the purification assembly 20. Specifically, the socket 43 includes a first plug fixing socket 431 and a second plug fixing socket 432, where the first plug fixing socket 431 is connected with the mains supply and the second plug fixing socket 432 is connected with a host machine of the range hood to supply power to the host machine of the range hood.

The electrical assembly 40 further includes a circuit board 44 connected with the first plug fixing socket 431 and the second plug fixing socket 432. Thus, the circuit board 44 can perform control and adjustment on electric energy such that the purification device 100 can work normally.

With reference to FIG. 3, in some embodiments, the electrical assembly 40 includes an air velocity sensor 45 disposed in the airflow channel 11. The air velocity sensor 45 can detect an airflow rate in the airflow channel 11 and the airflow rate in the airflow channel 11 can be adjusted based on data obtained by the air velocity sensor 45.

With reference to FIGS. 3 and 4, in some embodiments, the housing 10 includes a housing body 14 and an air inlet casing 15. The air inlet casing 15 is mounted at a lower part of the housing body 14. The air outlet holes 13 and a part of the airflow channel 11 are disposed in the housing body 14 and the air inlet holes 12 and a part of the airflow channel 11 are disposed in the air inlet casing 15. The ozone generation assemblies 22 are mounted inside the air inlet casing 15.

The disposal of the ozone generation assemblies 22 in the air inlet casing 15 facilitates the mounting of the ozone generation assemblies 22. Specifically, a grid structure is formed in the air inlet casing 15 to allow an air flow to run through the grid structure into the air inlet casing 15. The air inlet casing 15 may be mounted on the housing body 14 by screws.

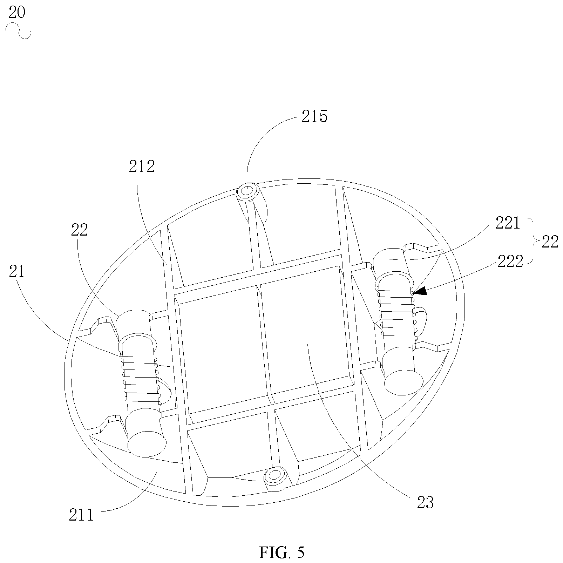

With reference to FIGS. 4 and 5, in some embodiments, the purification assembly 20 includes a cover plate 21 and an accommodation space 30 is formed between the cover plate 21 and the air inlet casing 15. The ozone generation assemblies 22 are located in the accommodation space 30 and the accommodation space 30 is in communication with the airflow channel 11.

When the ozone generation assemblies 22 work, the generated ozone will not overflow due to the coverage of the cover plate 21, and thus control on the generated ozone amount can be realized, avoiding the harm of a large amount of overflowing ozone to human body.

The cover plate 21 may be made of plastic. In some embodiments, the cover plate 21 may be made of quartz material. The specific material of the cover plate 21 is not limited herein. The cover plate 21 may be bowl-shaped and the ozone generation assemblies 22 are fixed on an inner wall 211 of the cover plate 21. The ozone generation assemblies 22 are connected with the housing 10. The ozone generation assemblies 22 are disposed between the cover plate 21 and the housing. Due to the blocking of the cover plate 21, the ozone generated during the operation of the ozone generation assemblies 22 will not easily overflow outside the purification assembly 20, protecting the users to some degree.

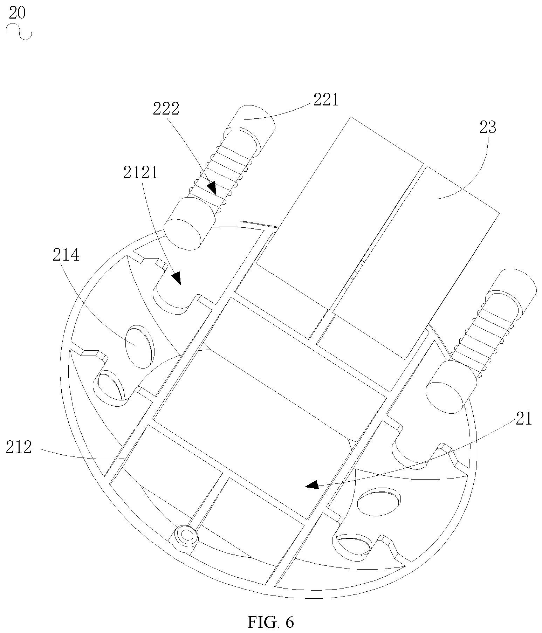

With reference to FIGS. 5 and 6, in some embodiments, plural partition strips 212 are disposed on the cover plate 21 and plural mounting holes 2121 are formed on the partition strips 212; each of the ozone generation assemblies 22 includes a mounting column 221 and a coil 222 surrounding the mounting column 221, and the mounting columns 221 are mounted on the mounting holes 2121. Thus, the mounting of the ozone generation assemblies 22 on the mounting holes 2121 makes the structure of the purification device 100 more compact, helping miniaturization of the purification device 100.

Specifically, plural partition strips 212 arranged on the inner wall 211 of the cover plate 21 can increase a rigidity of the cover plate 21. Plural turns of coil 222 may be wound on each of the mounting columns 221. The mounting columns 221 may be cylindrical. Plural mounting holes 2121 may be disposed. In one example, the mounting holes 2121 may be formed on each of two opposed partition strips 212. Along an axial direction of each mounting column 221, both ends of the mounting column 221 may be respectively fitted into two mounting holes 2121, so as to limit the mounting columns 221 and prevent the falling-off of the ozone generation assemblies 22.

With reference to FIGS. 5 and 6, in some embodiments, plural partition strips 212 and the cover plate 21 form a mounting groove, and the purification assembly 20 includes a transformer 23 electrically connected with the ozone generation assemblies 22, and the transformer 23 is disposed in the mounting groove. The mounting of the transformer 23 into the mounting groove of the cover plate 21 makes the structure more compact and helps miniaturization of the purification assembly 20.

In addition to mounting and fixing the ozone generation assemblies 22 and increasing the rigidity of the cover plate 21, the partition strips 212 can also form the mounting groove for mounting the transformer 23 together with the inner wall 211 of the cover plate 21. The mounting groove may be rectangular or circular or the like, which is not limited herein. In one example, the transformer 23 may be fixed to the inner wall 211 of the cover plate 21 by bonding to prevent the transformer 23 from slipping off the mounting groove and hence affecting the working efficiency of the purification device 100. It can be understood that the transformer 23 may also be connected with the mounting groove of the cover plate 21 in a fitting way. The mounting manner of the transformer 23 and the cover plate 21 is not limited herein.

More specifically, the transformer 23 may be electrically connected with the ozone generation assemblies 22 by wire. In this way, the most common standard voltage of the residents can be transformed and transmitted by the transformer 23 to the ozone generation assemblies 22. Thus, the ozone generation assemblies 22 form a working voltage to generate ozone. By changing a turn ratio of the inductance coils 222 in the transformer 23, the purpose of voltage transformation can be achieved. In some embodiments, an outer layer of the transformer 23 may be covered with resin to protect the transformer 23, thereby increasing the use performance of the transformer 23.

With reference to FIGS. 5 and 6, in one example, two ozone generation assemblies 22 are disposed, and respectively located at two opposed sides of the transformer 23. The two ozone generation assemblies 22 are respectively disposed at the two opposed sides of the transformer 23 such that ozone generated by the two ozone generation assemblies 22 is uniformly distributed.

Specifically, the ozone generation assemblies 22 and the transformer 23 are separated by the partition strips 212 such that the purification assembly 20 entirely looks aesthetic and compact, helping miniaturization of the purification device 100. In some embodiments, three or four or more ozone generation assemblies 22 may be disposed, which is not limited herein. A plurality of ozone generation assemblies 22 may be arranged around the transformer 23. The plurality of ozone generation assemblies 22 can increase the efficiency of the purification assembly 20 for ionizing air to generate ozone, so as to increase the purification capability of the purification device 100 for the fumes.

With reference to FIGS. 5 and 6, in some embodiments, through holes 214 in communication with the accommodation space 30 are formed on the cover plate 21. Thus, the ozone generated by the purification assembly 20 can flow out of the through holes 214 to perform sterilization, disinfection and deodorization on the fumes.

Specifically, a plurality of spaced through holes 214 may be formed on the cover plate 21, and air can flow through the through holes 214 on the cover plate 21 into the purification assembly 20, and the ozone generation assemblies 22 can ionize the air into ozone and the ozone then flows out of the through holes 214 to perform purification on the fumes.

With reference to FIGS. 5 and 6, in some embodiments, along a depth direction of the through holes 214, the through holes 214 are aligned with the corresponding ozone generation assemblies 22. Therefore, the ozone generated by the ozone generation assemblies 22 upon being powered can quickly run through the through holes 214 to perform purification on the fumes, so as to increase the purification efficiency of the purification assembly 20 for the fumes.

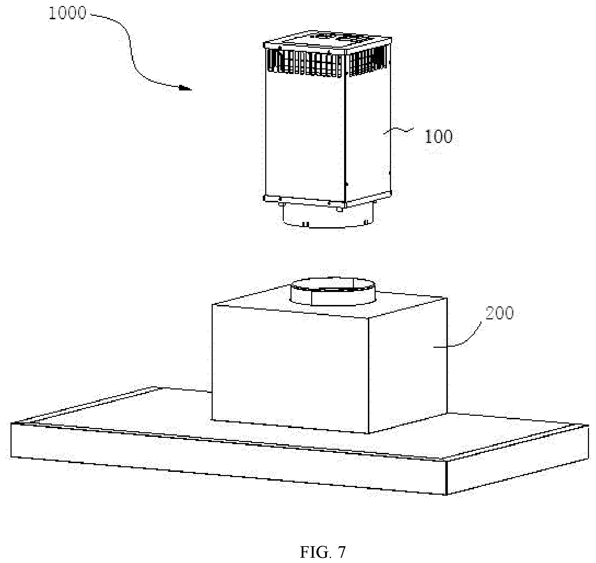

With reference to FIG. 7, the range hood 1000 provided by the embodiments of the present disclosure includes a host machine 200, the above purification device 100 and a fan (not shown). The purification device 100 is mounted on the host machine 200. The fan 300 is connected to the purification device 100 to suck gases and discharge the gases into the purification device 100.

In one example, the fan may be mounted in the host machine 200. The range hood 1000 may be disposed above countertop, and when a user uses the countertop to cook foods, the range hood 1000 can suck fumes and the like to prevent the fumes and the like from spreading in the whole kitchen, increasing the user experiences.

In the descriptions of the present disclosure, the descriptions made by referring to the terms "one embodiment", "some embodiments", "illustrative embodiments", "examples", "specific examples", or "some examples" or the like are meant to refer to that the specific features, structures, materials or characteristics described in combination with the embodiments or examples are included in at least one embodiment or example of the present disclosure. In the present disclosure, the illustrative expressions of the above terms do not necessarily refer to same embodiments or examples. Furthermore, the described specific features, structures, materials or characteristics can be combined in proper way in any one or more embodiments or examples.

Although the embodiments of the present disclosure have been illustrated and described, persons of ordinary skill in the arts can understand that various changes, modifications, replacements and variations may be made to these embodiments without departing from the principle and tenet of the present disclosure, and the scope of protection of the present disclosure is defined by the claims and its equivalents.

## Claims

1. A purification device, comprising:
a housing, wherein the housing is provided with an airflow channel, air inlet holes and air outlet holes, the air inlet holes are disposed at a lower part of the housing, the air outlet holes are disposed at an upper part of the housing, and the airflow channel communicates the air inlet holes with the air outlet holes;
a purification assembly, wherein the purification assembly comprises ozone generation assemblies and ultraviolet lamps, the ozone generation assemblies and the ultraviolet lamps are sequentially arranged in the airflow channel along an airflow direction of the airflow channel, and the ozone generation assemblies are used to generate ozone;
an electrical assembly, wherein the electrical assembly is mounted at the upper part of the housing, the electrical assembly comprises lamp holders, the ultraviolet lamps are mounted on the lamp holders, and the electrical assembly is electrically connected with the ozone generation assemblies.

2. The purification device of claim 1, wherein an opening is disposed on the top of the housing, and the electrical assembly comprises a mounting casing which partially protrudes into the airflow channel and closes the opening; the lamp holders are mounted on the mounting casing.

3. The purification device of claim 2, wherein a mounting space isolated from the airflow channel is formed in the mounting casing, the lamp holders are mounted in the mounting space, and the ultraviolet lamps protrude into the mounting space from the airflow channel and are connected with the lamp holders.

4. The purification device of claim 3, wherein the mounting casing comprises a first casing and a second casing, the first casing and the second casing are detachably connected and enclosed to form the mounting space, and the first casing is located above the second casing.

5. The purification device of claim 4, wherein the electrical assembly comprises a socket on the first casing and the socket is exposed on the top of the purification device.

6. The purification device of claim 1, wherein the housing comprises a housing body and an air inlet casing, the air inlet casing is mounted at a lower part of the housing body, the air outlet holes and a part of the airflow channel are disposed in the housing body, the air inlet holes and a part of the airflow channel are disposed in the air inlet casing, and the ozone generation assemblies are mounted inside the air inlet casing.

7. The purification device of claim 6, wherein the purification assembly comprises a cover plate, an accommodation space is formed between the cover plate and the air inlet casing, the ozone generation assemblies are located in the accommodation space, and the accommodation space is in communication with the airflow channel.

8. The purification device of claim 7, wherein plural partition strips are disposed on the cover plate, mounting holes are formed on the partition strips, each of the ozone generation assemblies comprises a mounting column and a coil surrounding the mounting column, and the mounting column is mounted on the mounting holes.

9. The purification device of claim 8, wherein the plural partition strips and the cover plate form a mounting groove, the purification assembly comprises a transformer electrically connected with the ozone generation assemblies, and the transformer is disposed inside the mounting groove.

10. A range hood, comprising:
a host machine;
the purification device of any one of claims 1 to 9, wherein the purification device is mounted on the host machine; and,
a fan connected with the purification device, wherein the fan is configured to suck gases and discharge the gases into the purification device.
